# EUROPEAN PATENT APPLICATION

(11) **EP 4 678 114 A1**
(43) Date of publication of application: **14.01.2026**
(21) Application number: 23926530.9
(22) Date of filing: 24.11.2023
(51) Int. Cl.: A61B 8/08

(54) **ULTRASOUND IMAGING SYSTEM AND ULTRASOUND IMAGING METHOD USING SAME**

(30) Priority: 09.03.2023 KR 20230031442; 27.09.2023 KR 20230130461
(71) Applicant: Center for Advanced Meta-Materials, Daejeon 34103 (KR)
(72) Inventor: KWEUN, Minwoo, Seoul 07404 (KR); LEE, Hak-Joo, Daejeon 35201 (KR); KIM, Jae-Hyun, Daejeon 34140 (KR); PIAO, Chun Guang, Daejeon 34155 (KR); JANG, Bongkyun, Daejeon 34103 (KR); KIM, Kwangseop, Daejeon 34140 (KR); LEE, Jaehwa, Daejeon 35226 (KR); CHOI, Hae Jin, Daejeon 34116 (KR)
(74) Representative: Lewis Silkin LLP
(86) International application number: PCT/KR2023/019063
(87) International publication number: WO 2024/185958

(57) **Abstract**

In an ultrasonic imaging system and an ultrasonic imaging method using the ultrasonic imaging system, the system includes a channel part, a correction part, an imaging part and a reference information providing part. The channel part has a plurality of channels configured to send and receive an ultrasonic signal. The correction part is configured to correct distortion caused by an ultrasonic barrier in the ultrasonic signal received by the channel part. The imaging part is configured to image the ultrasonic signal to an ultrasonic image. The reference information providing part is configured to provide reference information required for the correction to the correction part. The correction part is configured to determine a sample section using the reference information, and to correct attenuation distortion and aberration distortion caused by the ultrasonic barrier based on a signal of the sample section.

## Description

### BACKGROUND OF THE INVENTION

### TECHNICAL FIELD

Exemplary embodiments of the present invention relate to an ultrasonic imaging system and an ultrasonic imaging method using the ultrasonic imaging system. More particularly, exemplary embodiments of the present invention relate to an ultrasonic imaging system and an ultrasonic imaging method using the ultrasonic imaging system, for high-resolution imaging of distorted objects beyond barriers that scatter or reflect ultrasonic.

### DISCUSSION OF THE RELATED ART

Ultrasonic imaging technology is widely used in the diagnosis of organs that do not have bone or gas as an ultrasonic barrier, such as liver, breast, and thyroid ultrasonic.

On the other hand, ultrasonic penetration is difficult, such as gases in the skull, ribs, respiratory tract, or digestive tract. In addition, organs such as the brain, heart, lungs, stomach, and intestines are surrounded by or adjacent to barriers that cause chaotic refraction and scattering of signals. Thus, in this case, ultrasonic imaging is difficult to use.

In particular, in the case of the brain protected by the skull, it is possible to obtain clear images only through a gap in the skull called the fontanelle during the neonatal period. In adults, it is very difficult to obtain brain ultrasonic images, so only cerebral blood flow ultrasonic is used on a limited basis.

In addition, when obtaining echocardiographic images, ultrasonic is transmitted and received through the rib gap to perform image diagnosis with low resolution in a limited image range. In the case of lungs, ultrasonic imaging diagnosis is impossible due to ribs and internal gas. Digestive organs such as the stomach and intestines also cannot be diagnosed with ultrasonic imaging due to internal gas.

When ultrasonic imaging diagnosis is not possible, imaging diagnostic equipment such as CT or MRI is used, but these imaging diagnostic devices cannot be used in emergency settings such as general ambulances or emergency rooms. That is, due to low mobility (CT, MRI), radiation hazard (CT), contrast agent toxicity (CT, MRI), and waiting for an appointment (MRI), there are problems that make it impossible to use it in emergency scenes that require quick response or in seriously ill patients that require continuous monitoring.

Related prior arts include Koran patent No. 10-2146374.

### SUMMARY

Exemplary embodiments of the present invention provide an ultrasonic imaging system and an ultrasonic imaging method using the ultrasonic imaging system, capable of high-resolution imaging of distorted objects by correcting signals distorted by ultrasonic barriers that scatter or refract ultrasonic waves.

In addition, exemplary embodiments of the present invention also provide an ultrasonic imaging system and an ultrasonic imaging method using the ultrasonic imaging system, capable of correcting attenuation distortion caused by the ultrasonic barrier by equalizing the signal amplitude of the sample section estimated to be the object signal.

In addition, exemplary embodiments of the present invention also provide an ultrasonic imaging system and an ultrasonic imaging method using the ultrasonic imaging system, capable of moving the ultrasonic signal extraction section for each channel to correct aberration distortion caused by the ultrasonic barrier.

In addition, exemplary embodiments of the present invention also provide an ultrasonic imaging system and an ultrasonic imaging method using the ultrasonic imaging system, capable of correcting scattering distortion by automatically moving the position of the channel section to a position with less scattering noise.

According to one aspect of the present invention, the ultrasonic imaging system includes a channel part, a correction part, an imaging part and a reference information providing part. The channel part has a plurality of channels configured to send and receive an ultrasonic signal. The correction part is configured to correct distortion caused by an ultrasonic barrier in the ultrasonic signal received by the channel part. The imaging part is configured to image the ultrasonic signal to an ultrasonic image. The reference information providing part is configured to provide reference information required for the correction to the correction part. The correction part is configured to determine a sample section using the reference information, and to correct attenuation distortion and aberration distortion caused by the ultrasonic barrier based on a signal of the sample section.

In an exemplary embodiment, the correction part may be configured to use the reference information to determine the sample section in which the ultrasonic signal reflected from an object is received, to separate the signal of the sample section from the ultrasonic signal received in the channel part, and then to equalize an amplitude of the separated ultrasonic signal to correct the attenuation distortion caused by the ultrasonic barrier, in correcting the attenuation distortion, and to extract a signal to be imaged by applying a sampling time delay for each channel and to correct the aberration distortion caused by the ultrasonic barrier, in correcting the aberration distortion.

In an exemplary embodiment, the correction part may be configured to determine an initial sampling time delay set at which the ultrasonic signal reflected from the object is predicted to be received, to set a plurality of sampling time delay sets within a certain range from the determined initial sampling time delay set, to select a preset sampling time delay set capable of generating an image with predetermined optimal image quality as an optimized sampling time delay set, and to extract the signal to be imaged according to the selected optimized sampling time delay set.

In an exemplary embodiment, the correction part may be configured to determine the initial sampling time delay set so that signals with the strongest signal strength are extracted from the ultrasonic signals received in the channel part.

In an exemplary embodiment, the reference information may include at least one of an ultrasonic speed in the ultrasonic barrier, an ultrasonic speed in a section except for the ultrasonic barrier, a thickness of the ultrasonic barrier, a distance to an object, a size of the object, and a distance between the objects.

In an exemplary embodiment, the reference information may include a reference ultrasonic signal for a reference object whose shape is already known, taken together with the object, or a reference image obtained by imaging the reference ultrasonic signal. The reference ultrasonic signal or the reference image may be obtained in the absence of the ultrasonic barrier.

In an exemplary embodiment, the correction part may be configured to determine the sample section, considering the time difference between the section in which the signal representing the reference object is received in the received ultrasonic signal and the section in which the signal representing the reference object is received in the reference ultrasonic signal, and the distance difference between the reference object and the object.

In an exemplary embodiment, the correction part may be configured to select the optimized sampling time delay set based on image quality among a plurality of sampling time delay sets. The optimized sampling time delay set based on the image quality may be a sampling time delay set that maximizes a brightness of the object, minimizes a size of a focal point, or generates an ultrasonic image with a shape most similar to the already known shape of the object.

In an exemplary embodiment, the correction part may be configured to select the sampling time delay set that minimizes a standard deviation or dispersion of intensity between pixels of the ultrasonic image due to the corrected ultrasonic signal among a plurality of sampling time delay sets and the corresponding reference image, as the optimized sampling time delay set.

According to another aspect of the present invention, the ultrasonic imaging method includes sending an ultrasonic signal to an ultrasonic barrier, receiving an ultrasonic signal distorted by the ultrasonic barrier, providing a reference information required for the correction, determining a sample section in which the ultrasonic signal reflected from an object is received, using the reference information, and correcting attenuation distortion caused by the ultrasonic barrier based on a signal of the sample section, correcting aberration distortion caused by the ultrasonic barrier based on a signal of the sample section, and imaging the corrected ultrasonic signal to an ultrasonic image.

In an exemplary embodiment, in the correcting attenuation distortion, the signal of the sample section may be separated from the ultrasonic signal received, and then an amplitude of the separated ultrasonic signal may be equalized to correct the attenuation distortion caused by the ultrasonic barrier. In the correcting aberration distortion, a signal to be imaged may be extracted by applying a sampling time delay for each channel, and the aberration distortion caused by the ultrasonic barrier may be corrected.

In an exemplary embodiment, the correcting attenuation distortion includes determining an initial sampling time delay set at which the ultrasonic signal reflected from the object is predicted to be received, setting a plurality of sampling time delay sets within a certain range from the determined initial sampling time delay set, selecting a preset sampling time delay set capable of generating an image with predetermined optimal image quality as an optimized sampling time delay set, and extracting the signal to be imaged according to the selected optimized sampling time delay set.

In an exemplary embodiment, in determining an initial sampling time delay set, the initial sampling time delay set may be determined so that signals with the strongest signal strength are extracted from the ultrasonic signals.

In an exemplary embodiment, the reference information may include at least one of an ultrasonic speed in the ultrasonic barrier, an ultrasonic speed in a section except for the ultrasonic barrier, a thickness of the ultrasonic barrier, a distance to an object, a size of the object, and a distance between the objects.

In an exemplary embodiment, in the providing a reference information, a reference object whose shape is already known, taken together with the object, may be detected, and a reference ultrasonic signal for the reference object or a reference image obtained by imaging the reference ultrasonic signal may be provided. The reference ultrasonic signal or the reference image may be obtained in the absence of the ultrasonic barrier.

In an exemplary embodiment, the sampling time delay set that minimizes a standard deviation or dispersion of intensity between pixels of the ultrasonic image due to the corrected ultrasonic signal and the corresponding reference image, may be selected as the optimized sampling time delay set.

In an exemplary embodiment, the optimized sampling time delay set may be a sample time delay set that maximizes a brightness of the object, minimizes a size of a focal point, or generates an ultrasonic image with a shape most similar to the already known shape of the object.

According to another aspect of the present invention, the ultrasonic imaging method includes sending an ultrasonic signal to an ultrasonic barrier, receiving an ultrasonic signal distorted by the ultrasonic barrier, calculating a signal to noise ratio of an object, decreasing the signal to noise ratio by changing a position of a probe, correcting attenuation distortion or aberration distortion caused by the ultrasonic barrier based on a signal of the sample section, and imaging the corrected ultrasonic signal to an ultrasonic image.

According to another aspect of the present invention, the ultrasonic imaging method includes sending an ultrasonic signal to an ultrasonic barrier, receiving an ultrasonic signal distorted by the ultrasonic barrier, correcting attenuation distortion or aberration distortion caused by the ultrasonic barrier based on a signal of the sample section, calculating a resolution condition of an object, applying an optimized time delay set for aberration correction to a signal transmission channel, to irradiate a newly beamformed ultrasonic wave, and imaging the corrected ultrasonic signal to an ultrasonic image.

In an exemplary embodiment, the ultrasonic imaging method may further include calculating a signal to noise ratio of the object, and decreasing the signal to noise ratio by changing a position of a probe.

According to some exemplary embodiments of the present invention, the position of the channel part is moved so that the signal distorted by the ultrasonic barrier contains as little scattering distortion as possible. The amplitude of the signal in which attenuation distortion occurs is scaled. An appropriate sampling time delay is applied to each channel of the signal in which aberration distortion occurs. Thus, Objects beyond the ultrasonic barrier may be imaged at high resolution.

In addition, the present inventions may be applied to a medical ultrasonic imaging device that transmits and receives ultrasonic waves inside barrier tissues that have non-uniform properties and shapes, such as bone tissue, soft tissue, gas, etc., causing aberration, attenuation, and scattering distortion of ultrasonic waves. Further, the present invention may be widely used in a variety of non-destructive testing industries that image by penetrating through barriers (composite materials, insulation materials, rust or sludge in pipes, etc.) that have solid (metal, ceramic, plastic, etc.) or fluid materials of various compositions using ultrasonic waves and cause aberration, attenuation, and scattering distortion of ultrasonic waves.

### BRIEF DESCRIPTION OF THE DRAWINGS

FIG. 1 is a block diagram illustrating an ultrasonic imaging system according to an example embodiment of the present invention;
FIG. 2 is a block diagram illustrating an ultrasonic imaging system according to another example embodiment of the present invention;
FIG. 3 is an image showing a position control method using a position control part of FIG. 2;
FIG. 4 is a flow chart showing an ultrasonic imaging method using the ultrasonic imaging system according to an example embodiment of the present invention;
FIG. 5 is a flow chart showing an attenuation distortion correction of FIG. 4;
FIG. 6 is a flow chart showing an aberration distortion correction of FIG. 4;
FIG. 7 is a flow chart showing an ultrasonic imaging method using the ultrasonic imaging system according to another example embodiment of the present invention;
FIG. 8A and FIG. 8B are an ultrasonic signal and an ultrasonic image of a blood vessel simulating tube obtained in the absence of an ultrasonic barrier, respectively.
FIGS. 9A and 9B are an ultrasonic signal and an ultrasonic image of the blood vessel simulation tube obtained in the presence of an ultrasonic barrier, respectively, for the blood vessel simulating tube of FIGS. 8A and 8B;
FIG. 10A and FIG. 10B are an ultrasonic signal and an ultrasonic image obtained by correcting the ultrasonic signal of FIGS. 9A and 9B using the ultrasonic imaging system of FIG. 1 or FIG. 2;
FIG. 11 is a flow chart showing an ultrasonic imaging method using the ultrasonic imaging system according to still another example embodiment of the present invention; and
FIG. 12 is a flow chart showing an ultrasonic imaging method using the ultrasonic imaging system according to still another example embodiment of the present invention.

### <Reference numerals>

| | | | |
|---|---|---|---|
| 10 : | channel part | 20 : | correction part |
| 30 : | imaging part | 40 : | reference information providing part |
| 50 : | position control part | 100, 100' : | ultrasonic imaging system |

### DETAILED DESCRIPTION OF THE INVENTION

Hereinafter, the present invention will be explained in detail with reference to the accompanying drawings.

The present invention is described more fully hereinafter with reference to the accompanying drawings, in which exemplary embodiments of the present invention are shown.

The present invention may, however, be embodied in many different forms and should not be construed as limited to the exemplary embodiments set forth herein. Rather, these exemplary embodiments are provided so that this disclosure will be thorough and complete, and will fully convey the scope of the present invention to those skilled in the art.

It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

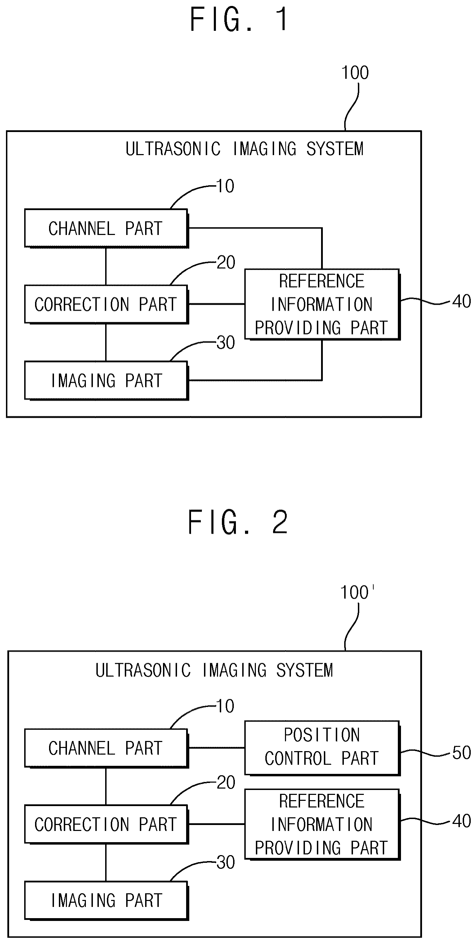

FIG. 1 is a block diagram illustrating an ultrasonic imaging system according to an example embodiment of the present invention.

The ultrasonic imaging system (hereinafter, the system) 100 includes a channel part 10, a correction part 20, an imaging part 30 and a reference information providing part 40.

The channel part 10 includes a plurality of channels (for example, a piezoelectric element), and is a transceiver configured to send and receive an ultrasonic signal.

Depending on the time delay applied to each channel, the channel part may transmit the ultrasonic signal in the form of a plane wave or divergent wave.

When the ultrasonic signal transmitted by the channel part propagates into a body, it is reflected at each point of a soft tissue with slightly different physical properties, and the reflected ultrasonic signal is received again by the channel part.

In general, when there is no barrier such as the skull, the ultrasonic signal having approximately the same intensity is received in the plurality of channels of the channel part. However, if ultrasonic waves pass through a barrier such as the skull, signal intensity between the channels is not consistent and attenuation distortion occurs, which reduces overall image brightness, due to non-uniform distribution of physical properties of the barrier, differences in acoustic impedance, and changes in the angle of incidence/penetration of transmitted and received ultrasonic waves, etc.

In addition, due to the non-uniform distribution of physical properties of barriers such as the skull, the signals reflected from the object do not enter each channel at the same time, resulting in aberration distortion in which the wavefronts of neighboring channels and signals are unevenly connected.

Thus, the system 100 of the present example embodiment corrects the ultrasonic signal received from the channel part 10, so that the above distortion phenomenon does not appear in the ultrasonic image.

Specifically, the correction part 20 performs attenuation distortion correction and aberration distortion correction before imaging an ultrasonic signal distorted by the barrier such as a skull.

The correction part 20 performs scaling correction so that the amplitude of the ultrasonic signal received for each channel becomes uniform, and thus, the attenuation distortion correction solves the problem that the signal intensity between the channels becomes inconsistent as the ultrasonic signal passes through the barrier.

At this time, if the amplitude is made uniform based on the signal of the entire time range coming into each channel, an accurate image may not be obtained.

Therefore, among the total signals coming into the channel, the section related to the region of interest (ROI), or more specifically, the section estimated to be the signal of the object within the region of interest, is determined as a sample section, and then based on the maximum amplitude or average amplitude of the sample section, the maximum amplitude or average amplitude of the ultrasonic signal received through each channel is scaled to be uniform.

Information for determining the sample section is received from the reference information providing part 40. The correction part 20 determines the sample section using the received reference information and then separates the ultrasonic signal corresponding to the sample section from the ultrasonic signal, and scales the maximum amplitude or average amplitude of the separated ultrasonic signals to become uniform.

Meanwhile, the aberration distortion correction is a correction to solve the problem that the signal arrival time between the channels is different as the ultrasonic signal passes through the barrier.

If there is no ultrasonic barrier, the ultrasonic signals reflected from the object are received almost simultaneously in the plurality of channels constituting the channel part. However, if there is the ultrasonic barrier such as the skull, the ultrasonic signal reflected from the object may be received with a time difference in each channel, and it is not possible to know exactly when the received signal is the ultrasonic signal reflected from the object.

Accordingly, the correction part 20 determines a sampling time delay set to move the section for extracting the signal intensity for each channel back and forth so that the wavefront of the signal with neighboring channels is smoothly connected, and the correction part 20 extracts signals to be used for imaging for each channel according to the determined sampling time delay set and transmits the extracted signals to the imaging part.

At this time, the correction part 20 may receive a corrected ultrasonic image obtained by imaging the ultrasonic signal transmitted to the imaging part, and may determine whether the corrected ultrasonic image satisfies a predetermined image quality standard.

The predetermined image quality standard may be whether to maximize the brightness of the object, minimize the size of the focal point, or generate an ultrasonic image with a shape most similar to the already known shape of the object. Additionally, the predetermined image quality standard may be whether an image similar to the ultrasonic image is generated by an undistorted ultrasonic signal.

If the corrected ultrasonic image does not meet predetermined criteria, the correction part 20 repeats updating the sampling time delay set until the corrected ultrasonic image satisfies a predetermined standard and transmitting the extracted signal to the imaging unit according to the updated sampling time delay set, and thus the ultrasonic images with minimal distortion caused by barriers may be provided.

In addition, the correction part 20 may determine the sampling time delay set using the reference information (e.g., a reference ultrasonic signal or reference image for a reference body) received from the reference information providing part 40. A detailed description of this will be described later.

The imaging part 30 images the ultrasonic signal to the ultrasonic image.

The imaging part 30 may image the ultrasonic signal received through the channel part 10 without correction to generate an uncorrected initial distorted image. This initially distorted image may be provided to the reference information providing part 40.

Additionally, the imaging part 30 may image the corrected ultrasonic signal provided by the correction part 20 to generate the ultrasonic image in which the aberration and attenuation distortion have been corrected.

Since the method of converting the ultrasonic signal into the ultrasonic image is a technology widely known to those skilled in the art, a detailed description will be omitted.

The reference information providing part 40 provides the correction part 20 with reference information for correcting the signals distorted by the barrier.

The reference information provided by the reference information providing part 40 may be information that may be used to predict when the received signal among the ultrasonic signals received through each channel is a signal reflected from an object.

For example, the ultrasonic speed at the ultrasonic barrier, the ultrasonic speed at a section other than the ultrasonic barrier, the thickness of the ultrasonic barrier, the distance to the object, the size of the object, the distance between the objects, etc. may be reference information.

Here, the ultrasonic speed may be a statistically known average speed, maximum speed, minimum speed, etc. Thickness/distance/size may be a statistically known average value, maximum value, minimum value, or a value predicted from the initial distorted image provided from the imaging part.

Additionally, the reference information may be a reference ultrasonic signal and/or a reference image for the reference object whose shape is already known.

Here, the reference object is photographed together with the object to be imaged and refers to an object whose shape is already known. For example, a pineal gland, a surgical wire, etc. may be the reference objects.

The reference ultrasonic signal provided by the reference information providing part 40 is the ultrasonic signal obtained for the reference object in the absence of the barrier, and the reference image is the image of the reference ultrasonic signal. Hereinafter, the ultrasonic signal for the reference object is referred to as the reference ultrasonic signal, and the ultrasonic image for the reference object is referred to as the reference image.

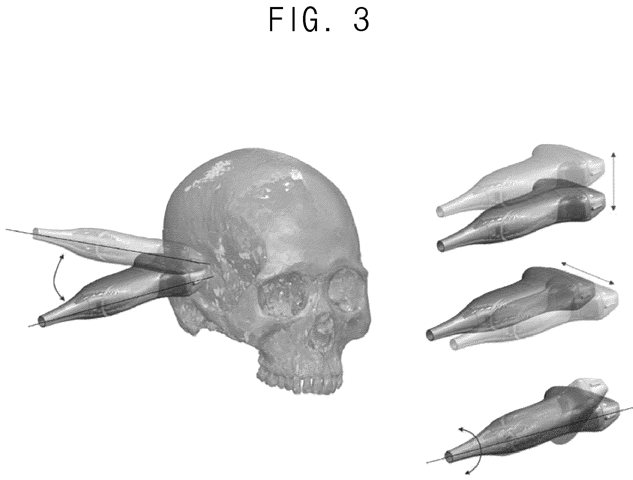

FIG. 2 is a block diagram illustrating an ultrasonic imaging system according to another example embodiment of the present invention. FIG. 3 is an image showing a position control method using a position control part of FIG. 2.

Referring to FIG. 2, the system 100' according to the present example embodiment includes a channel part 10, a correction part 20, an imaging part 30, a reference information providing part 40 and a position control part 50.

The system 100' according to the present example embodiment is substantially same as the system 100 of FIG. 1, except for the position control part 50, and thus scattering distortion may be further corrected.

Here, the scattering distortion refers to noise generated in the ultrasonic image by signals scattered/reflected by the barrier.

More specifically, part of the ultrasonic signal transmitted from the channel part 10 may be scattered while passing through the barrier and may be reflected multiple times within the barrier. Some of these scattered or reflected signals may accidentally be incident into the channel part at a similar time as the signal echoed from the object, and this becomes noise that degrades the image quality of the ultrasonic image.

The noise caused by such scattering distortion occurs frequently at the suture line where bone fragments of the skull meet, where the thickness is thick, and where there is a lot of porous tissue.

Thus, in the system 100' according to the present example embodiment, if the image quality of the acquired image does not meet predetermined standards, as illustrated in FIG. 3, by adjusting the angle and/or position of the channel part 10, the position control part 50 may time-gate the arrival time of the scattering signal from the arrival time of the image signal, thereby removing the noise caused by the scattering distortion.

As illustrated in FIG. 3, the position control part 50 adjusts the position or angle of the channel part 10 in the horizontal direction, vertical direction, and various other rotation directions, as shown by the arrows, to prevent the scattering distortion, and thus the noise caused by the scattering distortion may be removed.

That is, when changing the angle and/or position of the channel part, the signal scattered/reflected by the barrier and the signal reflected from the object travel a different path than before changing the angle and/or position of the channel part and then do not enter the channel part at similar times.

Therefore, the signal echoed from the object and the signal scattered/reflected from the barrier are temporally separated, and the noise caused by the scattering distortion may be removed.

As a predetermined standard for changing the angle and/or position of the channel part, the indicator that quantifies the intensity of the received ultrasonic signal and the degree of distortion of the wavefront or that quantifies image quality, such as brightness, spatial resolution, and contrast resolution of the imaged object, may be used. In addition, the position control part 50 adjusts the angle and/or position of the channel part 10 until the image quality satisfies predetermined standards, and then the system 100' according to the present example embodiment may provide clear ultrasonic image with the noise caused by the scattering distortion removed.

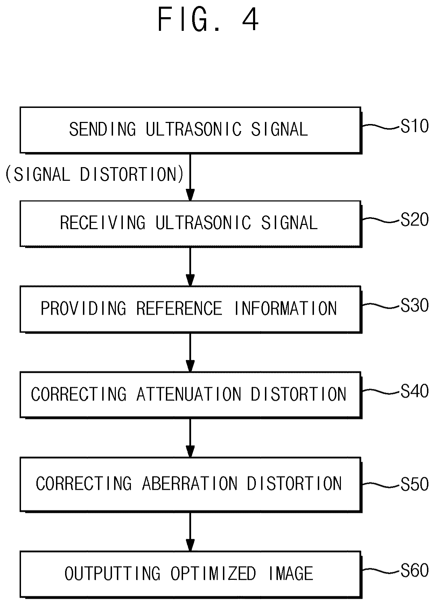

Hereinafter, an ultrasonic imaging method is explained referring to FIG. 4 to FIG. 6.

FIG. 4 is a flow chart showing an ultrasonic imaging method using the ultrasonic imaging system according to an example embodiment of the present invention.

Referring to FIG. 4, the ultrasonic imaging method (hereinafter, method) includes a step of sending ultrasonic signal (step S10). a step of receiving ultrasonic signal (Step S20), a step of providing reference information (step S30), a step of correcting attenuation distortion (step S40), a step of correcting aberration distortion (step S50), and a step of outputting optimized image (step S60).

The step of sending ultrasonic signal (step S10) is a step of transmitting the ultrasonic signal to the object from the channel part 10. At this time, depending on the time delay applied to the plurality of the channels constituting the channel part, the channel part may transmit the ultrasonic signal in the form of a plane wave or divergent wave.

The step of receiving ultrasonic signal (Step S20) is a step where the ultrasonic signal transmitted from the channel part goes through scattering, reflection, etc. and then reenters the channel part.

The received ultrasonic signal may be a signal containing noise due to the attenuation distortion, the aberration distortion, or the scattering distortion. Alternatively, the received ultrasonic signal may be a signal in which the noise due to the scattering distortion has been removed by position adjustment by the position control part 50, as in the system according to the example embodiment of FIG. 2.

The step of providing reference information (step S30) is a step of providing the reference information needed for the attenuation distortion correction and/or the aberration distortion correction by the correction part 20.

The reference information may be information that may be used to predict when, among ultrasonic signals received through each channel, the received signal is a signal reflected from the object.

For example, the highest ultrasonic speed in the ultrasonic barrier, the lowest ultrasonic speed, the average ultrasonic speed, the highest ultrasonic speed in a section other than the ultrasonic barrier, the lowest ultrasonic speed, the average ultrasonic speed, the maximum thickness of the ultrasonic barrier, the minimum thickness, the average thickness, the distance to the object, the size of the object, the distance between objects, etc. may be the reference information.

The step of correcting attenuation distortion (step S40) is a step of correcting the attenuation distortion by equalizing the amplitude of the ultrasonic signal received for each channel.

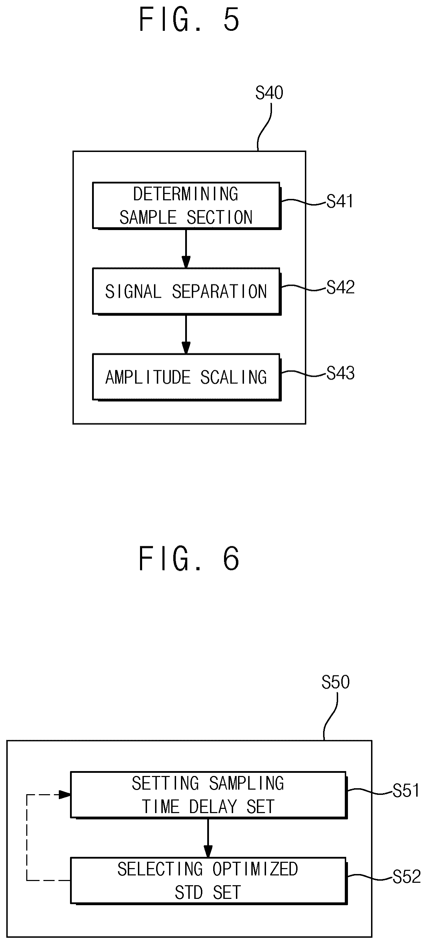

FIG. 5 is a flow chart showing an attenuation distortion correction of FIG. 4, and as illustrated in FIG. 5, the step of correcting attenuation distortion (step S40) includes a step of determining sample section (step S41), a step of separating signal (step S42) and a step of scaling amplitude (step S43).

More specifically, the correction part 20 uses the reference information provided by the reference information providing part 40 to determine the sample section, which is a time range in which the ultrasonic signal reflected from the object may be incident into the channel unit 10 (step S41).

Then, only signals in the sample section are separated from the ultrasonic signals received in each channel (step S42). For example, if the sample period is determined to be 100 to 170 microseconds (the ultrasonic frequency is 3 MHz and the object is approximately 75 mm away from the probe), the ultrasonic signals received in each channel at that time are separated.

Then, the amplitude of the separated ultrasonic signals is made to be uniform (step S43).

For example, the maximum amplitude or average amplitude of the ultrasonic signal received by each channel in the sample period is checked and scaled so that the maximum amplitude or average amplitude of the ultrasonic signal received by each channel in the sample period is constant.

By making the amplitude of the sample section uniform in the above way, the problem of the signal intensity between the channels becoming inconsistent as the ultrasonic signal passes through the ultrasonic barrier may be solved.

The step of correcting aberration distortion (step S50) is a step of differentiating the signal extraction section for each channel so that the wavefront of the signal with neighboring channels is smoothly connected to correct distortion caused by different signal arrival times between the channels as the ultrasonic signal passes through the ultrasonic barrier.

FIG. 6 is a flow chart showing an aberration distortion correction of FIG. 4, and as illustrated in FIG. 6, the step of correcting aberration distortion (step S50) includes a step of setting STD (sampling time delay) set (step S51), and a step of selecting optimized STD set (step S52).

In the step of setting STD (sampling time delay) set (step S51), the correction part 20 may set a plurality of STD sets within a certain range (for example, a number of wavelengths such as 0 to 2 wavelengths), based on an initial STD set from which signals estimated to be reflected from the object may be extracted. At this time, one STD set includes STDs to be applied to each channel (if there are N channels, one STD set includes N STDs), and the STD set may be set for each object or segmented ROI if there are multiple objects within the ROI or if the ROI is segmented.

For example, the correction part 20 may determine the time at which the signal reflected from the object was received using reference information (for example, ultrasonic maximum speed in the ultrasonic barrier, ultrasonic minimum speed, ultrasonic average speed, ultrasonic maximum speed in a section other than the ultrasonic barrier, ultrasonic minimum speed, ultrasonic average speed, maximum thickness of the ultrasonic barrier, minimum thickness, average thickness, distance to the object, size of the object, distance between the objects, etc.) provided from the reference information providing part 40 and determine the initial STD set using the determined time.

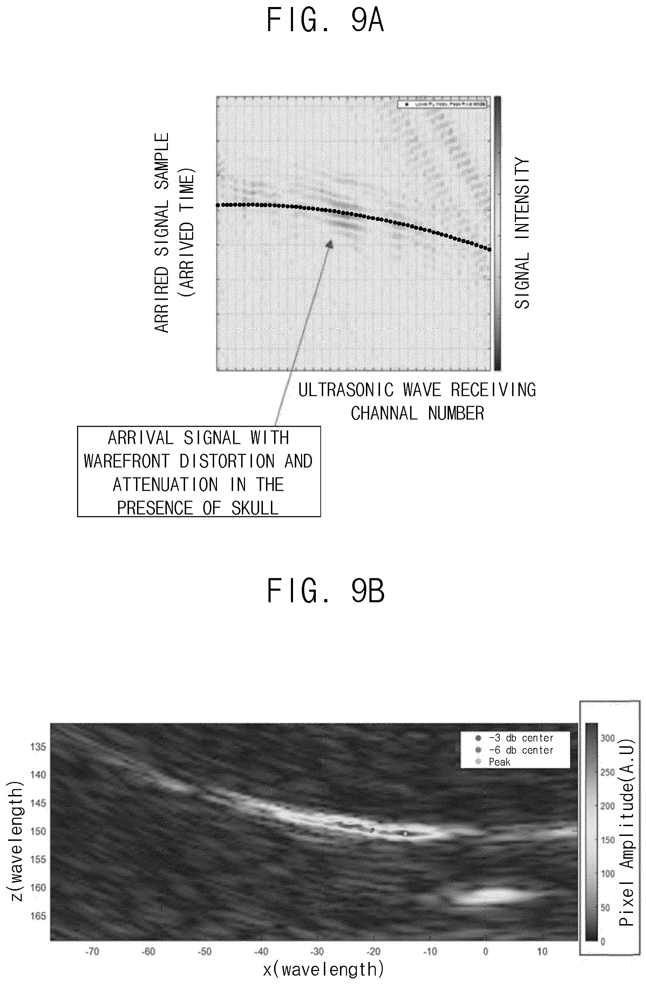

Additionally, the correction part 20 may determine the initial STD set so that the signals with the strongest signal intensity are extracted from the signals received for each channel (uncorrected signals or signals with attenuation distortion corrected). For example, the initial STD set may be determined so that the signals shown as black dots in the received ultrasonic signal as shown in FIG. 9A and the signals forming peaks in the ultrasonic image as shown in FIG. 9B are extracted.

Then, a certain section from the initial STD set may be set as the sampling time delay selection section, and the multiple STD sets may be set in that section. The STD selection section may be a smaller section than the sample section to match the signal amplitude in the above attenuation distortion. For example, the sample section selected to correct the amplitude of the attenuation distortion is the signal section where 1300 to 2000 signal samples are received. However, the STD selection section for the aberration correction is the section corresponding to the location of the specific object, has a curved shape for each channel, and refers to the section within several wavelengths (a signal section where several to dozens of signal samples are received).

The step of selecting optimized STD set (step S52) is a step of selecting the STD set that may generate images with optimal image quality among the plurality of STD sets.

The correction part 20 may select the optimized STD set using a heuristic algorithm, such as a genetic algorithm or particle swarm optimization algorithm, or a sensitivity or gradient-based algorithm. At this time, the optimized STD set is the STD set that may smoothly connect the wavefronts of neighboring channels and signals. An image with optimal image quality may be the image that maximizes the brightness of the object, minimizes the size of the focal point (e.g. size from the center of pixels above -6dB based on pressure), or is similar to the shape of a known object (e.g. -6dB pixel shape).

As illustrated in FIG. 6, the step of selecting optimized STD set (step S52) is processed after the step of setting the STD (sampling time delay) set (step S51). However, as indicated by the dashed arrow, in order to select the STD set with optimal image quality, the step of setting the sampling time delay set (step S51) may be returned according to the image quality evaluation results. In this case, a new STD set may be selected by reflecting the image quality evaluation results.

Referring to FIG. 4 again, after the above correcting processes, in the step of outputting optimized image (step S60), an ultrasonic image with the noise caused by the aberration distortion and the attenuation distortion is removed is outputted.

Although FIG. 4 shows that the aberration distortion is corrected after the attenuation distortion correction, it does not exclude that the attenuation distortion correction is performed after the aberration distortion correction.

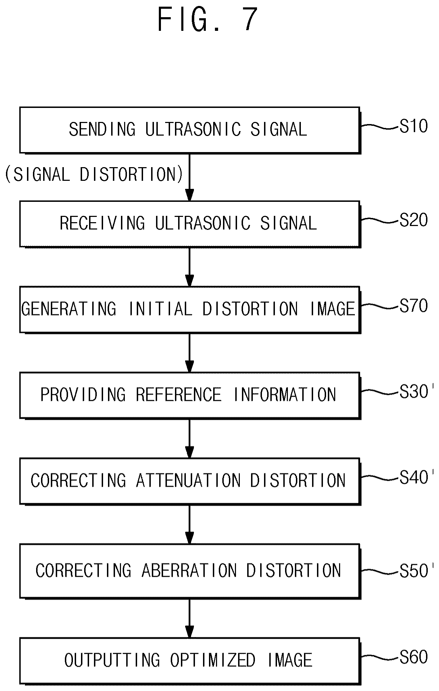

FIG. 7 is a flow chart showing an ultrasonic imaging method using the ultrasonic imaging system according to another example embodiment of the present invention.

As illustrated in FIG. 7, the method according to the present example embodiment includes a step of sending ultrasonic signal (step S10). a step of receiving ultrasonic signal (Step S20), a step of generating initial distortion image (step S70), a step of providing reference information (step S30'), a step of correcting attenuation distortion (step S40'), a step of correcting aberration distortion (step S50'), and a step of outputting optimized image (step S60).

The method according to the present example embodiment is substantially same as the method of FIG. 4, except for further including the step of generating initial distortion image (step S70).

Thus, in the method according to the present example embodiment, the explanation for the same steps in the method of FIG. 4 is omitted. Thus, the description will focus on differences from the method of FIG. 4 by further including the step of generating initial distortion image (step S70).

The step of generating initial distortion image (step S70) is a step of imaging the ultrasonic signal received in the channel part, that is, the ultrasonic signal in which distortion due to the barrier has not been corrected, into the ultrasonic image. That is, the initial distorted image is the ultrasound image generated from the ultrasonic signal containing noise due to distortion caused by the barrier.

The imaging part 30 may generate an initial distorted image and then provide the initial distorted image and/or the uncorrected ultrasonic signal to the reference information providing part 40 and/or the correction part 20 for the distortion correction.

In the step of providing reference information (step S30'), the reference information providing part 40 may provide the reference information. Here, for example, the highest ultrasonic speed in the ultrasonic barrier, the lowest ultrasonic speed, the average ultrasonic speed, the highest ultrasonic speed in a section other than the ultrasonic barrier, the lowest ultrasonic speed, the average ultrasonic speed, the maximum thickness of the ultrasonic barrier, the minimum thickness, the average thickness, the distance to the object, the size of the object, the distance between objects, etc. may be the reference information.

Additionally, the reference information providing part 40 identifies a reference object in the initial distorted image and provides a reference ultrasonic signal and/or a reference ultrasonic image to the correction part 20. At this time, the reference ultrasonic signal and/or the reference ultrasonic image may be information pre-stored in the reference information providing part 40.

In the step of correcting attenuation distortion (step S40'), the correction part 20 receiving the reference ultrasonic signal and/or the reference ultrasonic image may determine a more accurate sample section for attenuation distortion correction using the reference ultrasound signal and/or the reference ultrasound image.

The correction part 20 may predict the time difference caused by the barrier by comparing the section in which the signal representing the reference object is received in the ultrasonic signal received from the channel part with the section in which the signal representing the reference object is received in the reference ultrasonic signal. In addition, by further considering the distance difference (or arrival time difference) between the reference object and the object, the sample section, which is the time range in which the ultrasonic signal reflected from the object may be incident into the channel unit 10, may be more accurately determined.

In addition, the change in signal intensity due to the barrier may be predicted by comparing the intensity of the signal representing the reference object and the reference ultrasonic signal in the ultrasonic signal received from the channel part. Accordingly, in the step of correcting attenuation distortion (S40'), by uniformly scaling the signal amplitude of the sample section, changes in the overall decrease or increase in signal intensity due to the barrier may be further corrected.

The, in the step of correcting aberration distortion (step S50'), the correction part 20 may determine a more optimized STD using a reference ultrasonic signal and/or a reference ultrasonic image.

The correction part 20 compares the section in which the signal representing the reference object is received from the ultrasonic signal received from the channel part with the section in which the signal representing the reference object is received in the reference ultrasonic signal, and then may predict the time difference caused by the barrier. This time difference or a time difference profile that interpolates the time difference for each channel may be determined as an initial sampling time delay (STD) set.

Then, by comparing the ultrasonic image imaged by applying the initial STD set and the reference image, the STD set is updated so that the standard deviation or variance of the intensity of the corresponding pixels of the two images is minimized, and thus the STD is optimized.

Specifically, to optimize by updating the STD set, the correction part 20 sets a certain section within several wavelengths from the initial STD set as the STD selection section, and sets multiple STD sets in the corresponding section, and then selects the STD set that may generate an image with optimal image quality as the optimal STD set. Here, an image with optimal image quality may be an image in which the standard deviation or dispersion of pixel intensity from the reference image is minimized. Here, the intensity of the pixel may be a brightness value, but is not limited thereto.

So far, the ultrasonic imaging system and ultrasonic imaging method have been described.

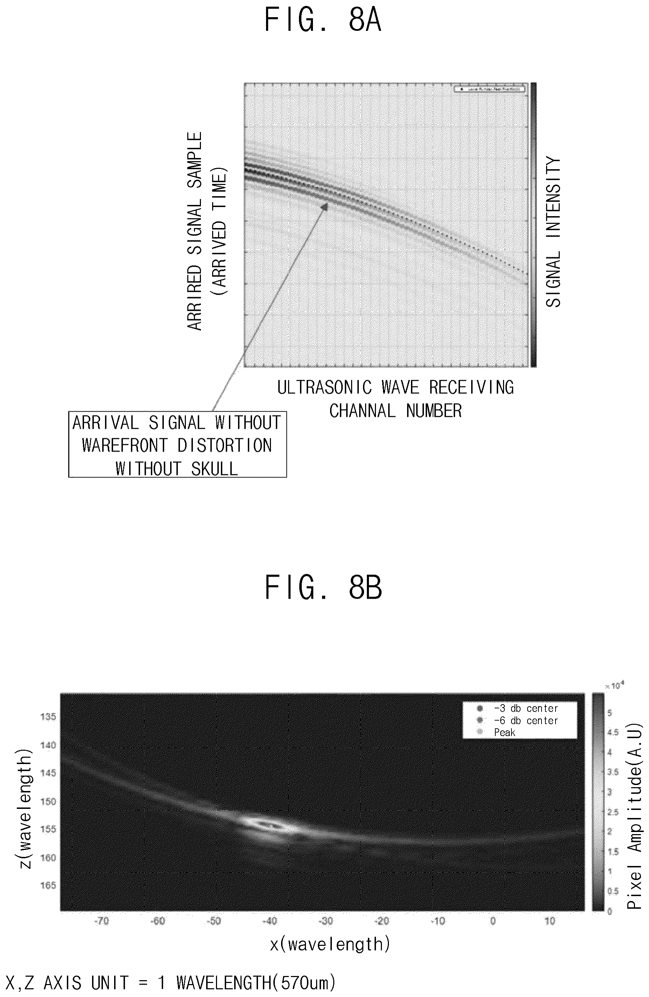

FIG. 8A and FIG. 8B show the ultrasonic signal and the ultrasonic image measured on a blood vessel simulating tube (2mm diameter, Teflon) when there is no ultrasonic barrier. FIG. 9A and FIG. 9B show the ultrasonic signal and the ultrasonic image measured through the same blood vessel simulation tube, when there is the ultrasonic barrier such as the skull. Referring to FIG. 8A, FIG. 8B, FIG. 9A and FIG. 9B, when there is the ultrasonic barrier, a lot of signal distortion occurs in the ultrasonic signal, which makes the ultrasonic image very different from the actual object (signal intensity decreases by about 170 times, horizontal tube size error occurs by about 400%, and horizontal tube position error occurs by about 50%).

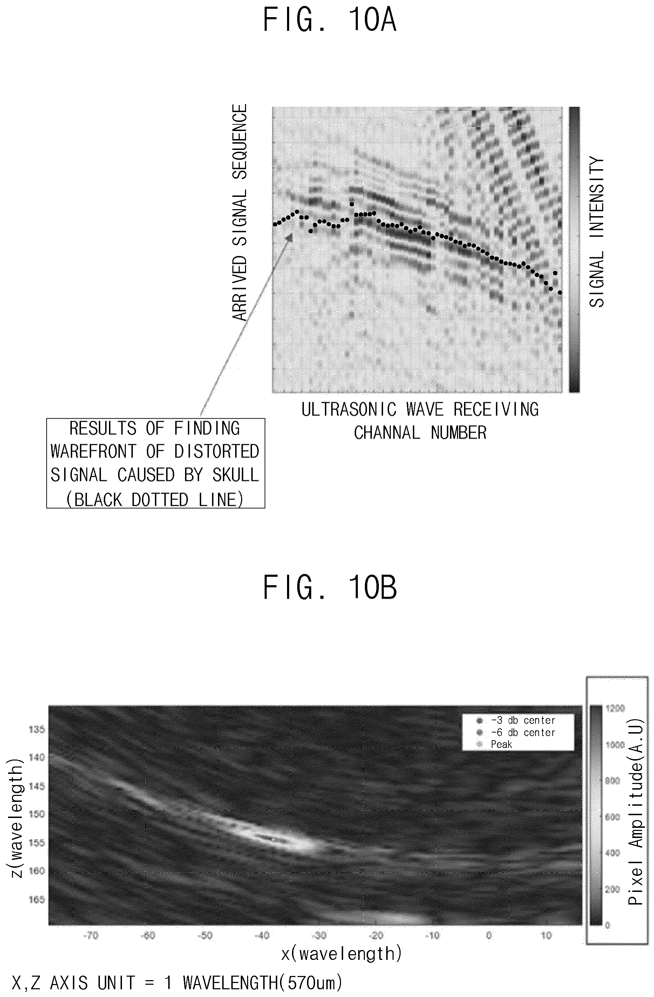

In the system and the method according to the present example embodiments, the distortion caused by the ultrasonic barrier may be corrected, using the attenuation distortion correction that uniformly scales the amplitude of the sample interval, the aberration distortion correction that adjusts the signal extraction section with STD optimized for each channel, and the scattering distortion correction that moves the channel position. Here, the ultrasonic image using the corrected ultrasonic signal may be corrected to closely resemble the actual object, as shown in FIG. 10A and FIG. 10B(signal intensity increased 4 times compared to before correction, tube size error decreased from 400% to 37%, tube position error decreased from 50% to 6.2%).

In the system and the method according to the present example embodiments, when using the reference ultrasonic signal and the reference ultrasonic image, an accurate ultrasonic image of the object may be obtained even if the step of sending ultrasonic signal and the step of receiving the ultrasonic signal are performed only once.

However, in the system and the method according to the present example embodiments, the step S10 and the step S20 are not limited to being performed only once, but may be performed more than twice.

When obtaining the ultrasonic signal from various angles by varying the steering angle of the channel part 10 and using them to obtain ultrasonic image of the object, spatial and contrast resolution may be improved.

At this time, if the optimized STD set of the i^{th} ultrasonic beam with an irradiation angle θ is used as the initial STD set for the (i+1)^{th} ultrasonic beam with an irradiation angle θ+dθ, the optimized STD set may be derived more quickly.

Also, in cases that the areas of the i^{th} ultrasonic beam and the (i+1)^{th} ultrasonic beam overlap, the optimal STD value may be derived more quickly by sharing the STD even if the ROI (or ultrasonic irradiation angle) is different, when the directions of the ultrasonic waves propagating in a specific channel K are close.

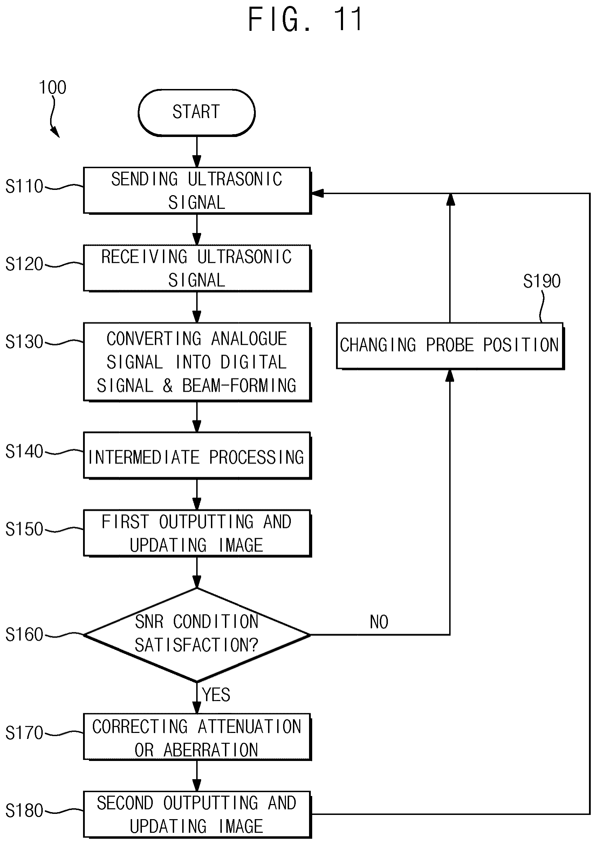

FIG. 11 is a flow chart showing an ultrasonic imaging method using the ultrasonic imaging system according to still another example embodiment of the present invention. FIG. 11 shows a method for restoring an image that controls the position of an ultrasonic probe and improves the signal-to-noise ratio of the object to facilitate subsequent attenuation and aberration distortion correction. The target signal that is recognized as noise relative to the target signal may be an electrical noise signal that stands out due to the scattering signal, echo signal, or attenuation of the porous tissue of the skull described above.

Referring to FIG. 11, the ultrasonic imaging method (step S100) according to the present example embodiment, includes a step of sending ultrasonic signal (step S110), a step of receiving ultrasonic signal (step S120), a step of converting the receiving analogue signal into a digital signal, a step of beam-forming (step S130) in which the signals for each receiving channel for differently transmitted ultrasonic are synthesized to correspond to the pixels of each image, a step of intermediate processing (step S140) in which efficient imaging is performed by extracting the phase and envelope of the signal or reducing the number of signal samples, to acquire B-mode images or Doppler signals in the future, a step of first outputting and updating image (step S150), a step of correcting the above-described attenuation or aberration (step S170) when the signal-to-noise ratio condition is satisfied (step S160), a step of finding an acoustic window to reduce the above-described noise by changing the probe position (step S190) when the signal-to-noise ratio condition is not satisfied (step S160), and a step of second outputting and updating image (step S180).

The step of converting the received analog signal into the digital signal, the step S130 and the step S140 are imaging methods of a general ultrasonic imaging device and may be basically included in the imaging process of the above-described embodiments. Additionally, the object and image in the present embodiment include B-mode or Doppler mode, which are general diagnostic modes.

In addition, in the present example embodiment, transmitting the ultrasonic wave is a method for generating a well-known compound image by irradiating the ultrasonic waves by varying the ultrasonic irradiation direction, focusing depth, or scan line. In addition, although not shown in the figure, it can also be applied to the process of transmitting several different ultrasonic waves toward the barrier as described above and correcting attenuation and aberration distortion for each received signal according to the transmission mode.

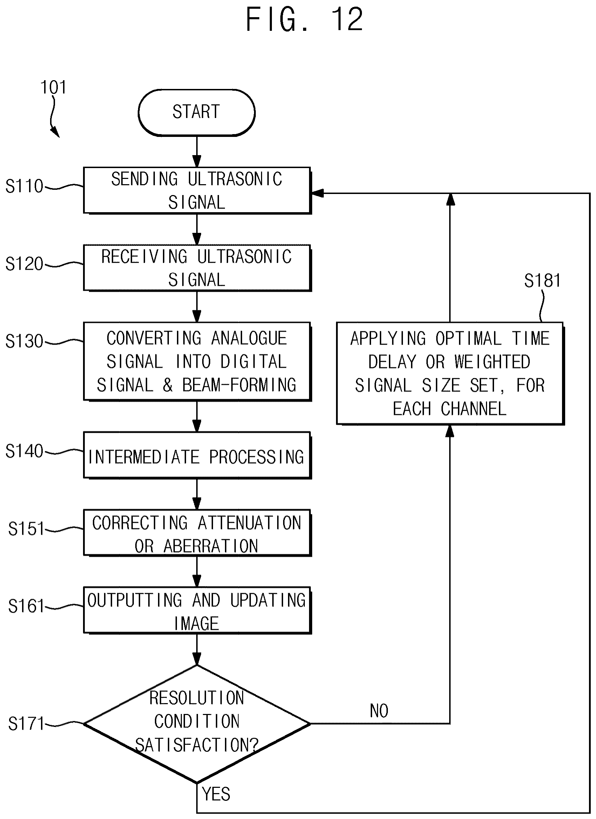

FIG. 12 is a flow chart showing an ultrasonic imaging method using the ultrasonic imaging system according to still another example embodiment of the present invention. FIG. 12 shows a method of applying the time delay set found by the aberration correction method described above to the beamforming signal of the ultrasonic transmission channel.

The ultrasonic imaging method (step S101) according to the present example embodiment, includes a step of sending ultrasonic signal (step S110), a step of receiving ultrasonic signal (step S120), a step of configuring initial image based on the receiving signal (a step of converting digital signal, a step of beam-forming (step S130), and a step of intermediate processing (step S140) are included in the step), a step of correcting attenuation distortion or aberration distortion caused by the ultrasonic barrier (step S151), a step of outputting image (step S161), a step of calculating resolution condition of the object based on the outputted image, and here if the resolution condition is not satisfied (step S171), a step of applying the optimal time delay set for the aberration correction or the weighted signal size set for each channel for the attenuation correction to the signal transmission channel to irradiate the newly beamformed ultrasonic wave (step S181).

At this time, if a time delay set or a weighted signal size for each channel is applied to beamforming of transmitted ultrasonic wave, the aberration or attenuation distortion of the object may be physically corrected. Then, the post-processing attenuation and aberration distortion correction process described above may be shortened and facilitated.

The above resolution condition may be the image brightness of the object, focal size, phase or amplitude deviation from the reference image, phase or amplitude deviation from the reference channel signal, time difference or amplitude deviation from the reference time delay set.

Furthermore, the feedback loop continues even if the resolution conditions of the object are met. This is a process in which the ultrasonic image is generally updated to a higher frame so that the movement of the object may be clearly observed.

In addition, although not shown, the ultrasonic probe position control method described in FIG. 11 and FIG. 12 and the method of applying the found time delay set to beamforming of transmitted ultrasonic waves may be combined. Here, the sequence may be one method is completed first and then the other method is performed, or the two methods may be performed together each time within a feedback loop that repeats in a series of sequences.

According to some exemplary embodiments of the present invention, the position of the channel part is moved so that the signal distorted by the ultrasonic barrier contains as little scattering distortion as possible. The amplitude of the signal in which attenuation distortion occurs is scaled. An appropriate sampling time delay is applied to each channel of the signal in which aberration distortion occurs. Thus, Objects beyond the ultrasonic barrier may be imaged at high resolution.

In addition, the present inventions may be applied to a medical ultrasonic imaging device that transmits and receives ultrasonic waves inside barrier tissues that have non-uniform properties and shapes, such as bone tissue, soft tissue, gas, etc., causing aberration, attenuation, and scattering distortion of ultrasonic waves. Further, the present invention may be widely used in a variety of non-destructive testing industries that image by penetrating through barriers (composite materials, insulation materials, rust or sludge in pipes, etc.) that have solid (metal, ceramic, plastic, etc.) or fluid materials of various compositions using ultrasonic waves and cause aberration, attenuation, and scattering distortion of ultrasonic waves.

Having described exemplary embodiments of the present invention, it is further noted that it is readily apparent to those of reasonable skill in the art that various modifications may be made without departing from the spirit and scope of the invention which is defined by the metes and bounds of the appended claims.

## Claims

1. An ultrasonic imaging system comprising:
a channel part having a plurality of channels configured to send and receive an ultrasonic signal;
a correction part configured to correct distortion caused by an ultrasonic barrier in the ultrasonic signal received by the channel part;
an imaging part configured to image the ultrasonic signal to an ultrasonic image; and
a reference information providing part configured to provide reference information required for the correction to the correction part,
wherein the correction part is configured to determine a sample section using the reference information, and to correct attenuation distortion and aberration distortion caused by the ultrasonic barrier based on a signal of the sample section.

2. The ultrasonic imaging system of claim 1, wherein the correction part is configured:
to use the reference information to determine the sample section in which the ultrasonic signal reflected from an object is received;
to separate the signal of the sample section from the ultrasonic signal received in the channel part, and then to equalize an amplitude of the separated ultrasonic signal to correct the attenuation distortion caused by the ultrasonic barrier, in correcting the attenuation distortion; and
to extract a signal to be imaged by applying a sampling time delay for each channel and to correct the aberration distortion caused by the ultrasonic barrier, in correcting the aberration distortion.

3. The ultrasonic imaging system of claim 2, wherein the correction part is configured:
to determine an initial sampling time delay set at which the ultrasonic signal reflected from the object is predicted to be received;
to set a plurality of sampling time delay sets within a certain range from the determined initial sampling time delay set;
to select a preset sampling time delay set capable of generating an image with predetermined optimal image quality as an optimized sampling time delay set; and
to extract the signal to be imaged according to the selected optimized sampling time delay set.

4. The ultrasonic imaging system of claim 3, wherein the correction part is configured to determine the initial sampling time delay set so that signals with the strongest signal strength are extracted from the ultrasonic signals received in the channel part.

5. The ultrasonic imaging system of one of claim 1 to claim 4, wherein the reference information comprises at least one of an ultrasonic speed in the ultrasonic barrier, an ultrasonic speed in a section except for the ultrasonic barrier, a thickness of the ultrasonic barrier, a distance to an object, a size of the object, and a distance between the objects.

6. The ultrasonic imaging system of claim 3, wherein the reference information comprises a reference ultrasonic signal for a reference object whose shape is already known, taken together with the object, or a reference image obtained by imaging the reference ultrasonic signal,
wherein the reference ultrasonic signal or the reference image is obtained in the absence of the ultrasonic barrier.

7. The ultrasonic imaging system of claim 6, wherein the correction part is configured to determine the sample section, considering the time difference between the section in which the signal representing the reference object is received in the received ultrasonic signal and the section in which the signal representing the reference object is received in the reference ultrasonic signal, and the distance difference between the reference object and the object.

8. The ultrasonic imaging system of claim 7, wherein the correction part is configured to select the optimized sampling time delay set based on image quality among a plurality of sampling time delay sets,
wherein the optimized sampling time delay set based on the image quality is a sampling time delay set that maximizes a brightness of the object, minimizes a size of a focal point, or generates an ultrasonic image with a shape most similar to the already known shape of the object.

9. The ultrasonic imaging system of claim 6, wherein the correction part is configured to select the sampling time delay set that minimizes a standard deviation or dispersion of intensity between pixels of the ultrasonic image due to the corrected ultrasonic signal among a plurality of sampling time delay sets and the corresponding reference image, as the optimized sampling time delay set.

10. An ultrasonic imaging method comprising:
sending an ultrasonic signal to an ultrasonic barrier;
receiving an ultrasonic signal distorted by the ultrasonic barrier;
providing reference information required for the correction;
determining a sample section in which the ultrasonic signal reflected from an object is received, using the reference information, and correcting attenuation distortion caused by the ultrasonic barrier based on a signal of the sample section;
correcting aberration distortion caused by the ultrasonic barrier based on a signal of the sample section; and
imaging the corrected ultrasonic signal to an ultrasonic image.

11. The ultrasonic imaging method of claim 10, wherein in the correcting attenuation distortion, the signal of the sample section is separated from the ultrasonic signal received, and then an amplitude of the separated ultrasonic signal is equalized to correct the attenuation distortion caused by the ultrasonic barrier,
wherein in the correcting aberration distortion, a signal to be imaged is extracted by applying a sampling time delay for each channel, and the aberration distortion caused by the ultrasonic barrier is corrected.

12. The ultrasonic imaging method of claim 11, wherein the correcting attenuation distortion comprises:
determining an initial sampling time delay set at which the ultrasonic signal reflected from the object is predicted to be received;
setting a plurality of sampling time delay sets within a certain range from the determined initial sampling time delay set;
selecting a preset sampling time delay set capable of generating an image with predetermined optimal image quality as an optimized sampling time delay set; and
extracting the signal to be imaged according to the selected optimized sampling time delay set.

13. The ultrasonic imaging method of claim 12, wherein in determining an initial sampling time delay set,
the initial sampling time delay set is determined so that signals with the strongest signal strength are extracted from the ultrasonic signals.

14. The ultrasonic imaging method of one of claim 10 to claim 13, wherein the reference information comprises at least one of an ultrasonic speed in the ultrasonic barrier, an ultrasonic speed in a section except for the ultrasonic barrier, a thickness of the ultrasonic barrier, a distance to an object, a size of the object, and a distance between the objects.

15. The ultrasonic imaging method of claim 12, wherein in the providing reference information,
a reference object whose shape is already known, taken together with the object, is detected, and a reference ultrasonic signal for the reference object or a reference image obtained by imaging the reference ultrasonic signal is provided,
wherein the reference ultrasonic signal or the reference image is obtained in the absence of the ultrasonic barrier.

16. The ultrasonic imaging method of claim 15, wherein the sampling time delay set that minimizes a standard deviation or dispersion of intensity between pixels of the ultrasonic image due to the corrected ultrasonic signal and the corresponding reference image, is selected as the optimized sampling time delay set.

17. The ultrasonic imaging method of claim 12, wherein the optimized sampling time delay set is a sample time delay set that maximizes a brightness of the object, minimizes a size of a focal point, or generates an ultrasonic image with a shape most similar to the already known shape of the object.

18. An ultrasonic imaging method comprising:
sending an ultrasonic signal to an ultrasonic barrier;
receiving an ultrasonic signal distorted by the ultrasonic barrier;
calculating a signal to noise ratio of an object;
decreasing the signal to noise ratio by changing a position of a probe;
correcting attenuation distortion or aberration distortion caused by the ultrasonic barrier based on a signal of the sample section; and
imaging the corrected ultrasonic signal to an ultrasonic image.

19. An ultrasonic imaging method comprising:
sending an ultrasonic signal to an ultrasonic barrier;
receiving an ultrasonic signal distorted by the ultrasonic barrier;
correcting attenuation distortion or aberration distortion caused by the ultrasonic barrier based on a signal of the sample section;
calculating a resolution condition of an object;
applying an optimized time delay set for aberration correction to a signal transmission channel, to irradiate a newly beamformed ultrasonic wave; and
imaging the corrected ultrasonic signal to an ultrasonic image.

20. The ultrasonic imaging method of claim 19, further comprising:
calculating a signal to noise ratio of the object; and
decreasing the signal to noise ratio by changing a position of a probe.
